# EUROPÄISCHE PATENTSCHRIFT

(11) **EP 1 583 525 B1**
(45) Veröffentlichungstag und Bekanntmachung des Hinweises auf die Patenterteilung: **01.09.2010**
(21) Anmeldenummer: 03815354.0
(22) Anmeldetag: 23.12.2003
(51) Int. Cl.: A61K 31/195, A61K 31/60, A61K 31/375, A61K 41/00, A61P 17/00, A61P 17/06, A61P 19/00, A61P 19/02, A61P 25/00

(54) **VERWENDUNG EINER KOMBINATION VON SUBSTANZEN DER PORPHYRINSYNTHESE, SALICYLATEN UND ANTIOXIDANTIEN BEI DER PHOTOTHERAPIE VON HAUT- UND/ODER GELENKERKRANKUNGEN**
USE OF PORPHYRIN SYNTHESIS SUBSTANCES IN COMBINATION WITH SALICYLATES AND ANTIOXIDANTS FOR USE IN PHOTOTHERAPY TO CURE SKIN AND ARTICULATION DISEASES
UTILISATION DE SUBSTANCES DE LA SYNTHESE DE PORPHYRINE EN COMBINAISON AVEC DES SALICYLATES ET DES ANTIOXIDANTS POUR L'UTILISATION EN PHOTOTHERAPIE POUR LE TRAITEMENT DE MALADIES DE LA PEAU ET DES ARTICULATIONS

(30) Priorität: 17.01.2003 DE 10301917
(43) Veröffentlichungstag der Anmeldung: 12.10.2005
(73) Patentinhaber: Saalmann, Gerhard, 32049 Herford (DE)
(72) Erfinder: SAALMANN, Gerhard, 32049 Herford (DE); SAALMANN, Peter, 32049 Herford (DE); TRONNIER, Hagen, 58313 Herdecke (DE)
(74) Vertreter: Miller, Andreas
(86) Internationale Anmeldenummer: PCT/DE2003/004254
(87) Internationale Veröffentlichungsnummer: WO 2004/064827

(56) Entgegenhaltungen:
- WO-A-00/02491
- WO-A-96/06602
- WO-A-96/28412
- WO-A-98/15273
- WO-A-02/100478
- WO-A-2004/024144
- DE-A- 10 003 620
- DE-A- 19 852 245
- US-A- 5 368 841
- US-A- 5 520 905
- CALZAVARA-PINTON P G ET AL: "PHOTODYNAMIC THERAPY WITH SYSTEMIC ADMINISTRATION OF PHOTOSENSITIZERS IN DERMATOLOGY" JOURNAL OF PHOTOCHEMISTRY AND PHOTOBIOLOGY B: BIOLOGY, ELSEVIER SCIENCE S.A., BASEL, CH, Bd. 36, Nr. 2, 1. November 1996 (1996-11-01), Seiten 225-231, XP000675962 ISSN: 1011-1344
- DRAKE L.A. ET AL: 'Guidelines of care for psoriasis' JOURNAL OF THE AMERICAN ACADEMY OF DERMATOLOGY Bd. 28, Nr. 4, 01 April 1993, C.V. MOSBY, ST. LOUIS, MO, US, Seiten 632 - 637, XP025849119
- PINNELL S.R.: 'Topical vitamin C: A new photo-protectant' JOURNAL OF THE EUROPEAN ACADEMY OF DERMATOLOGY AND VENEREOLOGY Bd. 11, 01 September 1998, ELSEVIER SCIENCE PUBLISHERS, AMSTERDAM, NL, Seite S167, XP004555645

## Beschreibung

Die Erfindung betrifft die Verwendung von Substanzen der Porphyrinsynthese in Kombination mit Salicylaten und Antioxidantien bei der Anwendung der Phototherapie zur Behandlung der Psoriasis oder entzündlicher Prozesse z. B. an der Haut und/oder den Gelenken von Säugetieren und Menschen.

Weiterhin betrifft die Erfindung die Verwendung von Substanzen der Porphyrinsynthese in Kombination mit Salicylaten und Antioxidantien zur Herstellung von Arzneimitteln zur Anwendung bei der Phototherapie bei der Behandlung der Psoriasis oder entzündlicher Prozesse z. B. an der Haut und/oder den Gelenken von Säugetieren und Menschen.

Unter entzündlichen Prozessen werden im Rahmen der Erfindung insbesondere die Psoriasis arthropathica und die nicht-psoriatische Polyarthritis verstanden.

Bei der Psoriasis arthropatica handelt es sich, anders als bei der nicht-psoriatischen Polyarthritis, wie der rheumatoiden Arthritis und ähnlichen Krankheitsbildern, um das Zusammentreffen einer Psoriasis vulgaris mit mono-oder polyarthritischen Gelenkveränderungen, die neben anderen Gelenken insbesondere die Finger-, Fuss-und Zehengelenke, sowie die Wirbelsäule und die Hüftgelenke betreffen. Im Gegensatz zur rheumatoiden Arthritis fallen bei Patienten mit Psoriasis arthropatica die rheuma- serologischen Tests normalerweise negativ aus. Zur Behandlung der psoriatischen Arthritis werden derzeit überwiegend nicht-steroidale Antiphlogistika, aber auch Goldpräparate, Glukokortikosteroide, Retinoide, sowie Methotrexat und Cyclosporine eingesetzt. Der Erfolg dieser medikamentösen Massnahmen ist jedoch oft unbefriedigend und insbesondere mit relativ hohen Nebenwirkungen bzw. Nebenwirkungsrisiken verbunden. Unerwünschte Nebenwirkungen sind besonders bei der meist erforderlichen Langzeittherapie zu beobachten.

Aufgabe der Erfindung ist es, solche Nebenwirkungen durch Bereitstellung und Verabreichung nebenwirkungsarmer Wirkstoffe bzw. Wirkstoffkombinationen in Verbindung mit einer nachfolgenden Bestrahlung mit sichtbarem Licht weitgehend zu vermeiden und gleichzeitig die Erfolgsrate und Verträglichkeit gegenüber den bekannten rein medikamentösen Behandlungsmethoden erheblich zu steigern.

Phototherapeutische Massnahmen in Kombination mit der Anwendung von Medikamenten sind bereits verschiedentlich bekannt geworden : So beschreibt die DE-A 100 63 076 die Verwendung von Aminolaevulinsäure zur Restenoseprophylaxe bei photodynamischer Therapie unter Anwendung von subletalen Lichtdosen.

Es ist auch bereits bekannt, dass bei der Behandlung von Hautkrankheiten verschiedene Wirkstoffe in Verbindung mit einer Bestrahlung des Körpers mit UV-Licht oder VIS wirksam werden (Psoriasis, Medizin in der Praxis, 20/00, S. 55-59).

Aus Mund-Kiefer-und Gesichtschirurgie, Abstract Volume 5, lssue 2 (2001) S. 98- 101, ISSN Nr. : 1432-9417, ist auch eine experimentelle 5-Aminolävulinsäure- induzierte photodynamische Therapie (ALA-PDT) zur Behandlung solider Tumoren bekannt geworden. Als Lichtquelle wurde Laserlicht einer Wellenlänge von 635 nm und einer Leistung von 0,75 Watt eingesetzt.

Das Dokument WO-A 96/06,602 offenbart eine pharmazeutische Zubereitung mit erhöhter Stabilität, die 5-Aminolaevulinsäure (5-ALA) oder pharmazeutische Äquivalente davon und einen pharmazeutisch annehmbaren Träger umfasst. Gegebenenfalls kann zusätzlich eine stabilisierende Menge eines organischen schwachen Protonen-Donors oder eine ein Saccharid enthaltende Substanz zugegen sein.

Weiter offenbart das Dokument DE-A 100 03 620 eine 5-Aminolaevulinsäure-(5-ALA-) Formulierung in nicht-wäßrigen Lösungsmitteln, umfassend als Wirksubstanz 5-ALA und/oder ein Derivat davon, gelöst oder dispergiert in einer nicht-wäßrigen Flüssigkeit mit einer Dielektrizitätskonstante ε < 80 (bei 25 °C). Weiter können als weitere Arzneistoffe solche Stoffe enthalten sein, die ausgewählt sein können aus Lokalanaesthetika, Antibiotika, Prostaglandinen, steroidalen und nicht-steroidalen Entzündungshemmern, Wachstumshormonen, Cytokinen wie etwa TNF, Sexualhormonen oder Vitaminen.

Weiterhin ist es bekannt, Aspirin zusammen mit nichtsteroidalen Antiphlogistika, wie z. B. Ibuprofen, zur Therapie von Arthritiden einzusetzen (Medications for Arthritis: www. orthop. washington. edu/arthritis/medications/05).

Zur Lösung der Aufgabe der Erfindung wird vorgeschlagen, Stoffe der Porphyrinsynthese, sowie von deren pharmakologisch brauchbaren Estern oder Salzen mit pharmakologisch verträglichen Säuren oder Basen, in Kombination mit Salicylaten, vorzugsweise Acetylsalicylsäure und verträglichen Antioxidantien, vorzugsweise Ascorbinsäure, bei der Bestrahlungstherapie mit Licht einer Wellenlänge von 400 bis 700 nm, vorzugsweise 520 bis 580 nm, insbesondere im Bereich um 545 nm, zur Behandlung der Psoriasis und/oder entzündlichen Verände- rungen an Gelenken des Menschen oder von Säugetieren, bereitzustellen und zu verwenden.

Die Methoden des oben angeführten Standes der Technik tragen zur vorgeschlagenen Lösung der Aufgabe der Erfindung nichts bei und eröffnen dem Fachmann keinen Weg, der Psoriasis oder entzündlichen Prozessen in den Gelenken wirklich erfolgreich und zuverlässig zu begegnen und gleichzeitig Nebenwirkungen weitestgehend auszuschliessen.

Gegenstand der Erfindung ist somit die in den Patentansprüchen näher bezeichnete Verwendung von Substanzen der Porphyrinsynthese, insbesondere der 5- Aminolaevulinsäure, in Kombination mit Salicylaten und Antioxidantien.

Die Erfindung betrifft also eine Kombination von mindestens einer Substanz der Porphyrinsynthese oder von deren pharmakologisch verträglichen Estern oder Salzen, mindestens eines Salicylats und mindestens eines Antioxidans bei der Anwendung einer Phototherapie zur Behandlung der Arthritis, Psoriasis vulgaris, Psoriasis arthropathica, sowie von Neuropathien wie dem Karpaltunnelsyndrom, oder dem Morbus Bechterew.

Eine bevorzugte Ausführungsform einer solchen Kombination ist im abhängigen Anspruch 2 beansprucht.

Weiter betrifft die Erfindung die Verwendung einer Kombination von mindestens einer Substanz der Porphyrinsynthese oder deren pharmakologisch verträglichen Estern oder Salzen, mindestens eines Salicylats und mindestens eines Antioxidans zur Herstellung eines topisch oder systemisch zu verabreichenden Arzneimittels für die Anwendung der Phototherapie mit einem Licht der Wellenlänge von 400 bis 700 nm zur Behandlung der Arthritis, Psoriasis vulgaris, Psoriasis arthropathica, sowie von Neuropathien wie dem Karpaltunnelsyndrom, oder dem Morbus Bechterew bei Säugetieren und Menschen.

Bevorzugte Ausführungsformen solcher Verwendungen sind in den abhängigen Ansprüchen 4 bis 7 beansprucht.

Die Substanzen der Porphyrinsynthese (vorzugsweise 5-Aminolävulinsäure, kurz: ALA) sowie deren pharmakologisch brauchbaren Derivate oder Salze werden in Kombination mit Salicylaten (vorzugsweise Acetylsalicylsäure) eingesetzt. Weiter werden sie zusätzlich kombiniert mit Antioxidantien, vorzugsweise Ascorbinsäure.

Die erfindungsgemässen Substanzen sind systemisch oder lokal, parenteral oder enteral, vorzugsweise oral oder topisch in Form üblicher Arzneimittelzubereitungen verabreichbar. Die jeweils gewählten Wirkstoffe, bzw. deren Kombination kann vom Patienten auf besonders einfache Weise peroral, beispielsweise in Wasser oder Fruchtsaft gelöst oder suspendiert, eingenommen werden. Insbesondere für lokale Behandlungen können spezielle Injektionsformen vorgesehen werden.

Bei lokaler Behandlung ist es vorteilhaft, die Wirkstoffe oder deren Kombination entweder topisch durch percutane Infiltration in das Gewebe der betroffenen Körperstellen zu verabreichen oder diese tiefer in das betroffene Gewebe zu injizieren. Somit kommen einmal percutane Applikationsformen, wie Salben, Cremes oder Lotionen und zum anderen für die parenterale Injektion geeignete sterile Lösungen oder Emulsionen in Frage. Bei Salbengrundlagen empfiehlt sich ein vor der Bestrahlung angelegter Okklusivverband, der die aufgetragenen Wirkstoffe einschliesst und damit sowohl die Wirksamkeit erhöht als auch die notwendige Einwirkungszeit verkürzt.

Als typische Arzneimittelzubereitungen kommen folglich alle üblichen pharmazeutischen Formen in Frage, die sich für eine parenterale oder enterale, insbesondere orale oder ggf. auch topische Verabreichung eignen. Dies sind z. B. Pulver, Tabletten, Dragees, Weich-oder Hartgelatinekapseln, Brausetabletten, Emulsionen, Öle, Lösungen oder Lyophilisate, sowie sterile Injektionslösungen oder Emulsionen mit üblichen Hilfs-und Zusatzstoffen.

Durch die neuartige Kombinationstherapie gemäss der vorliegenden Erfindung erreicht man überraschend eine zumindest teilweise, oft aber auch eine vollständige Reduzierung von akuten oder chronischen, spezifischen oder unspezifischen Gelenkentzündungen, sowie den Abbau von Bewegungseinschränkungen, eine weitgehende Schmerzfreiheit und die Rückführung von Schwellungen bis zum Normalzustand der befallenen Regionen des Körpers.

Desgleichen ist die erfindungsgemäss vorgeschlagene Therapie bei der Behandlung der Psoriasis der Haut in ihren unterschiedlichen klinischen Formen besonders wirksam. Die neuartige Kombination der Gabe von nebenwirkungsarmen Wirkstoffen mit einer Bestrahlung mit Licht eines definierten Wellenlängenbereichs von 400 bis 700 nm, vorzugsweise 520 bis 580 nm, insbesondere im Bereich um 545 nm, ist insbesondere bei der Behandlung der Psoriasis arthropatica (Psoriasis arthritis), von Arthritisformen anderer Pathogenese, von Neuropathien (z. B. dem Karpaltunnelsyndrom) sowie der Spondylarthritis antryloetica (Morbus Bechterew) angezeigt.

Unter Stoffen der Porphyrinsynthese werden erfindungsgemäss insbesondere die 5-Aminolaevulinsäure (ALA) oder deren Ester, wie z. B. der Methylester (MALA) oder Salze, insbesondere die Hydrochloride, verstanden.

Allgemein sind alle Stoffe brauchbar, die während der Behandlung im menschlichen oder tierischen Gewebe zu Protoporphyrin IX (PP IX) verstoffwechselt werden, denn PP IX ist bei der Bestrahlung der wirksame Photosensibilisator; dieser wird dann im Organismus weiter zu Häm umgesetzt.

Unter dem Begriff Ester werden im Rahmen der vorliegenden Erfindung allgemein die Ester von Carboxylgruppen der eingesetzten Wirkstoffe mit gesättigten oder ungesättigten, geradkettigen oder verzweigten C1 bis C4-aliphatischen oder C3- bis C7- cycloaliphatischen Alkoholen oder sonstigen unbedenklichen oder die Therapie unterstützenden Verbindungen, die eine alkoholische OH-Gruppe aufweisen, verstanden. Umgekehrt sind natürlich auch Ester mit alkoholischen OH-Gruppen der Wirkstoffe denkbar, die dann mit pharmakologisch unbedenklichen Säuren, wie z. B. Essigsäure oder Propionsäure gebildet werden.

Zu den Alkoholen zählen insbesondere C1- bis C4- aliphatische Alkohole wie Methanol, Ethanol, Propanol und Isopropanol.

Als Salzkomponenten mit basischen oder sauren Gruppen der erfindungsgemäss verwendeten Substanzen werden im Rahmen der Erfindung Salze mit pharmakologisch verträglichen anorganischen oder organischen Säuren oder Basen verstanden.

Hierzu gehören z. B. Hydrochloride, Hydrobromide, sowie analog die Sulfate, Phosphate, Nitrate, Acetate, Propionate, Citrate, Lactate, Mandelate, Sorbate, Ascorbate oder die Maleate.

Mit sauren Gruppen, insbesondere Carboxylgruppen, der Wirkstoffe erhält man brauchbare Lithium-, Natrium-, Kalium-, Calcium-, Magnesium- oder Zinksalze, sowie quaternäre Ammoniumsalze mit Ammoniak oder aliphatischen Aminen wie z. B. Methylamin oder Ethylamin. Es versteht sich, dass hier ausserdem eine grosse Zahl weiterer Salzkomponenten in Frage kommt, wie sie bereits weitläufig pharmazeutisch zur Anwendung gelangt und bekannt sind.

Als Salicylat ist besonders die nebenwirkungsarme Acetylsalicylsäure einsetzbar. Daneben kommen auch Salicylsäure selbst oder andere wirksame Salicylsäurederivate oder deren Salze in Frage, wie z. B. Natriumsalicylat, Methylsalicylat oder Hydroxyethylsalicylat.

Als Antioxidantien können alle pharmakologisch unbedenklichen und ggf. die Therapie unterstützenden Verbindungen mit einem ausreichenden Redoxpotential, insbesondere die Ascorbinsäure, eingesetzt werden. Hierzu zählen z. B. auch folgende Substanzen, bzw. deren Salze oder Derivate : Isoascorbinsäure, Tocopherol, Gluconsäure oder Karotinoide.

Bewährt hat sich eine Kombination der 5-Aminolaevulinsäure mit Acetylsalicylsäure und Ascorbinsäure im Gewichtsverhältnis von etwa 1 : 3 : 2.

Der Therapieablauf besteht aus der parenteralen oder enteralen, insbesondere oralen oder topischen Gabe der erfindungsgemässen Zubereitungen gefolgt von einer Wartezeit von 60 bis 180, vorzugsweise 150 Minuten und einer sich anschliessenden Phototherapie mit nicht zytotoxisch wirksamen Bestrahlungsdosen im oben angegebenen Wellenlängenbereich. Ausser der Ganzkörperbestrahlung können besonders wirksam auch Teilpartien und Einzelgelenke bestrahlt werden. Ebenso ist es möglich, über Lichtleiter oder Endoskope das Licht direkt an das entzündete Gewebe heran zu bringen.

Als nicht zytotoxisch gilt eine wirksame Strahlungsdosis im Bereich von etwa 5 bis 50 J/cm2. Sie muss je nach Empfindlichkeit des Patienten so gewählt werden, dass an den bestrahlten Körperregionen erkennbare und unerwünschte Begleiterscheinungen, wie z. B. Hautreizungen oder Entzündungserscheinungen vermieden werden. Da die Therapie in der Regel aus mehreren, vorzugsweise 6 bis 15 Bestrahlungen besteht, ist es dem behandelnden Arzt leicht möglich, sich an die optimale Bestrahlungsdosis heranzutasten und so Überdosierungen zu vermeiden. Da die Bestrahlung mit dem wenig aggressiven sichtbaren Licht erfolgt, ist die Behandlung im Gegensatz zu ultraviolettem Licht ohnehin in sehr weiten Grenzen unproblematisch.

Die Belichtungs- und Bestrahlungseinheit kann aus einer oder mehreren Lampeneinheiten bestehen, durch die die Haut ganz oder teilweise mit sichtbarem Licht des oben angegebenen Wellenlängenbereichs, ganz besonders bevorzugt mit Grünlicht einer Wellenlänge von 540 bis 550 nm, bestrahlt wird. Die Intensität der Behandlung wird je nach Konstitution des Patienten und der Dauer und Ernsthaftigkeit seiner Erkrankung durch Variation der Wirkstoffe, der Bestrahlungsstärke, der Wellenlänge, des Bestrahlungsabstands, der Bestrahlungsdauer und, bei wiederholten Behandlungen, des Zeitabstands der Bestrahlungen gesteuert. Die erforderliche Bestrahlungsdosis bzw. die Bestrahlungsdauer ist vom Arzt anhand der obigen Kriterien und der speziellen Anamnese ohne weiteres ermittelbar.

Erfindungsgemäss wird für die Ganzkörperbestrahlung eine Bestrahlungsdosis von 5 bis 50 J/cm2 vorgeschlagen. Bevorzugt ist eine Bestrahlungsdosis von etwa 15 J/ cm2. Bei lokaler Behandlung empfiehlt sich eine Bestrahlungsdosis von 10-80 J/ cm2 Die Bestrahlungsdauer ist abhängig vom Abstand der Strahlungsquelle von der zu bestrahlenden Körperoberfläche und der Strahlungsleistung der verwendeten Strahler. Im Normalfall sollen die Lichtquellen bei Ganzkörperbestrahlung einen Abstand von 10 bis 50 cm haben. Bei einer Strahlungsleistung von 20 mW/cm2 be- trägt die Bestrahlungszeit pro Behandlung etwa 20 bis 30 Minuten.

Bei lokaler Behandlung beträgt der Abstand eines Strahlers mit einer Leistung von 40 mW/cm2 von der Oberfläche des zu behandelnden Körperteils etwa 10 bis 15 cm. Die Bestrahlungsdauer liegt in diesem Fall zwischen 10 und 20 Minuten. Die angegebenen Parameter sind am Normalfall ausgerichtet und können im Rahmen der Verträglichkeit durchaus hiervon abweichen.

Untersuchungsergebnisse Im Rahmen einer Pilotstudie sind 5 Patienten mit der Diagnose einer schweren Psoriasis arthropatica behandelt worden.

Die Ergebnisse werden wie folgt zusammengefasst:

| Geschlecht | ØAlter (Jahre) | ØKrankheitsdauer (Jahre) | bisherige Behandlung |
|---|---|---|---|
| m : 1 | 50,4 | 16,4 | MTX, Kortikoide, |
| w : 4 | | | nicht steroidale Anti-phlogistica |

Ergebnisse nach dreiwöchiger Therapie (9 Anwendungen in gleichen Abständen)

| | | |
|---|---|---|
| Sehr gut: | 4 | (erscheinungsfrei) |
| Gut : | 1 | (geringe Schmerzen und Mobilitätseinschränkungen) |
| Mässig oder ohne Besserung : | 0 | |

### Fallbeispiel

Ein 41 Jahre alter Mann mit einem Körpergewicht von 80 kg litt seit seit 15 Jahren an erythemato-squamöser Psoriasis v. der Prädilektionsstellen und seit 5 Jahren an Psoriasis arthritis der Interphalangealgelenke der Hände und Füsse. Er klagte über Bewegungseinschränkung, Morgensteifigkeit und Druckschmerz. Die bisherige Therapie bestand aus der Verabreichung nichtsteroidaler Antiphlogistica und dem Antirheumaticum Methotrexat in einer Dosierung von 15 mg/Woche. Der Behandlungserfolg war mässig.

Zwei Wochen nach Absetzen der bisherigen Therapie wurde über einen Zeitraum von drei Wochen dreimal wöchentlich eine Kombination von 160 mg (2 mg/kg Körpergewicht) 5-Aminolävulinsäure, 400 mg (5 mg/kg Körpergewicht) Acetylsalicylsäure und 240 mg (3 mg/kg Körpergewicht) Ascorbinsäure oral verabreicht. Jeweils nach 150 Minuten Wartezeit nach der Applikation wurde eine Ganzkörperbestrahlung mit grünem Licht (Wellenlänge 540-550 nm, Dosis: 15 J/cm2) vorgenommen. Das Ergebnis der Behandlung war gut. Sowohl die Morgensteifigkeit als auch die Schmerzen nahmen erheblich ab. Gegenüber dem Resultat der Vorbehandlung wurde ohne subjektive Nebenwirkungen eine deutliche Abnahme der subjektiven und objektiven Symptomatik festgestellt. Das Arthritis-Score (Besserung in %) lag bei 56, dasjenige der Morgensteifigkeit bei 83 %. Die Laborwerte (Transaminasen, Blutbild, BSG) blieben unverändert.

## Patentansprüche

1. Kombination von mindestens einer Substanz der Porphyrinsynthese oder von deren pharmakologisch verträglichen Estern oder Salzen, mindestens eines Salicylats und mindestens eines Antioxidans bei der Anwendung einer Phototherapie zur Behandlung der Arthritis, Psoriasis vulgaris, Psoriasis arthropathica, sowie von Neuropathien wie dem Karpaltunnelsyndrom, oder dem Morbus Bechterew.

2. Kombination nach Anspruch 1, worin die mindestens eine Substanz der Porphyrinsynthese 5-Aminolaevulinsäure oder deren Ester oder deren Salz ist.

3. Verwendung einer Kombination von mindestens einer Substanz der Porphyrinsynthese oder deren pharmakologisch verträglichen Estern oder Salzen, mindestens eines Salieylats und mindestens eines Antioxidans zur Herstellung eines topisch oder systemisch zu verabreichenden Arzneimittels für die Anwendung der Phototherapie mit einem Licht der Wellenlänge von 400 bis 700 nm zur Behandlung der Arthritis, Psoriasis vulgaris, Psoriasis arthropathica, sowie von Neuropathien wie dem Karpaltunnelsyndrom, oder dem Morbus Bechterew bei Säugetieren und Menschen.

4. Verwendung nach Anspruch 3, worin die mindestens eine Substanz der Porphyrinsynthese 5-Aminolaevulinsäure oder deren Ester oder deren Salz ist.

5. Verwendung nach einem der Ansprüche 3 oder 4, worin das Salicylat Acetylsalicylsäure ist.

6. Verwendung nach einem der Ansprüche 3 bis 5, worin das Antioxidans Ascorbinsäure oder ein pharmakologisch verträgliches Salz derselben ist.

7. Verwendung nach einem der Ansprüche 3 bis 6, worin die Wellenlänge des Lichts um 545 nm liegt.

## Claims

1. Combination of at least one substance of the porphyrin synthesis or of their pharmacologically compatible esters or salts, at least one salicylate and at least one anti-oxidant when apply a phototherapy for the treatment of arthritis, *Psoriasis vulgaris, Psoriasis arthropathica* as well as of neuropathies, such as the carpal tunnel syndrome or Bechterew's disease.

2. Combination as set forth in claim 1 **characterised in that** the combination of at least one substance of the porphyrin consists of synthesis of 5-aminolaevulin acid or its ester or its salt.

3. Use of a combination of at least one substance of the porphyrin synthesis or of their pharmacologically compatible esters or salts, at least one salicylate and at least one anti-oxidant for the production of a topically or systemically applied pharmaceutical preparation for the application of the phototherapy with a light of the wave length of 400 to 700 nm for the treatment of arthritis, *Psoriasis vulgaris, Psoriasis arthropathica* as well as of neuropathies, such as the carpel tunnel syndrome or Bechterew's disease in mammals and humans.

4. Use as set forth in claim (3) **characterised in that** the combination of at least one substance of the porphyrin consists of synthesis of 5-aminolaevulin acid or its ester or its salt.

5. Use as set forth in claim (3) or (4) **characterised in that** the combination consists of the salicylate of acetylsalicylic acid.

6. Use as set forth in claims (3) to (5) **characterised in that** the combination consists of the anti-oxidant of ascorbic acid or a pharmacologically compatible acid of the said.

7. Use as set forth in claims (3) to (6) **characterised in that** the wave length of the light is around 545 nm.

## Revendications

1. Combinaison d'au moins une substance de la synthèse de la porphyrine ou de ses esters et sels compatibles d'un point de vue pharmacologique, au moins un salicylate et au moins un antioxydant lors de l'utilisation d'une photothérapie pour le traitement de l'arthrite, du psoriasis vulgaris, du psoriasis arthropathique ainsi que des neuropathies comme le syndrome du tunnel de Carpal ou de la spondylarthrite ankylosante.

2. Combinaison selon la revendication 1, au moins une substance de la synthèse de la porphyrine étant de l'acide aminolevunilique ou un des ses esters ou sels.

3. Utilisation d'une combinaison d'au moins une substance de la synthèse de la pophyrine ou de ses esters ou sels compatibles d'un point de vue pharmacologique, au moins un salicylate et au moins un antioxydant pour la fabrication d'un médicament à administrer de manière topique ou systémique pour l'application de la photothérapie avec une lumière de longueur d'onde comprise entre 400 et 700 nm pour le traitement de l'arthrose, du psoriasis vulgaris, du psoriasis arthropathique ainsi que des neuropathies comme le syndrome du tunnel de Carpal ou de la spondylarthrite ankylosante chez l'homme ou les mammifères.

4. Utilisation selon la revendication 3, au moins une substance de la synthèse de la porphyrine étant de l'acide aminolevunilique ou un des ses esters ou sels.

5. Utilisation selon une des revendications 3 ou 4, le salicylate étant de l'acide acétylsalicylique.

6. Utilisation selon une des revendications 3 à 5, l'antioxydant étant l'acide ascorbique ou un sel compatible d'un point de vue pharmacologique.

7. Utilisation selon une des revendications 3 à 6, la longueur d'onde de la lumière étant de 545 nm.
